# EUROPEAN PATENT SPECIFICATION

(11) **EP 0 649 467 B1**
(45) Date of publication and mention of the grant of the patent: **16.09.1998**
(21) Application number: 93918146.7
(22) Date of filing: 02.07.1993
(51) Int. Cl.: C12N 15/11, C07H 21/00, A61K 31/70

(54) **SELF-STABILIZED OLIGONUCLEOTIDES AS THERAPEUTIC AGENTS**
SELBSTSTABILISIERTE OLIGONUKLEOTIDE ALS THERAPEUTIKA
OLIGONUCLEOTIDES AUTO-STABILISES UTILES COMME AGENTS THERAPEUTIQUES

(30) Priority: 02.07.1992 US 909069
(43) Date of publication of application: 26.04.1995
(73) Proprietor: HYBRIDON, Inc., Worcester, Massachusetts 01605 (US)
(72) Inventor: AGRAWAL, Sudhir, Shrewsbury, MA 01545 (US); TANG, Jin-Yan, Worcester, MA 06104 (US)
(74) Representative: Vossius, Volker, Dr.
(86) International application number: US9306326
(87) International publication number: WO9401550

(56) References cited:
- WO-A-92/03464
- EMBO JOURNAL vol. 8, no. 13 , 1989 , EYNSHAM, OXFORD GB pages 4297 - 4305 CASE C.C. ET AL 'The unusual stability of the IS10 anti-sense RNA is critical for its function and is determined by the structure of the stem-domain'
- NUCLEIC ACIDS RESEARCH vol. 21, no. 11 , 11 June 1993 , ARLINGTON, VIRGINIA US pages 2729 - 2735 TANG J.Y. ET AL 'Self-stabilized antisense oligodeoxynucleotide phosphorothioates: properties and anti-HIV activity'

## Description

### BACKGROUND OF THE INVENTION

### 1. Field of the Invention

The invention relates to novel therapeutic agents used in the antisense oligonucleotide therapeutic approach. More particularly, the invention relates to improved antisense oligonucleotides that have increased resistance to nucleases.

### 2. Summary of the Related Art

The antisense oligonucleotide based therapeutic principle provides an attractive strategy for rationally designing antiviral drugs and chemotherapeutic agents against other pathogens, and against disease conditions resulting from disorders of gene expression. The therapeutic principle relies upon specific binding between a target nucleic acid sequence and a complementary oligonucleotide. Several publications have demonstrated the efficacy of complementary oligonucleotides in inhibiting gene expression by such specific binding.

Zamecnik and Stephenson, Proc. Natl. Acad. Sci. USA 75: 285-288 (1978) discloses specific inhibition of Rous Sarcoma Virus replication in infected chicken fibroblasts by a 13-mer synthetic oligodeoxynucleotide that is complementary to part of the viral genome.

Zamecnik et al., Proc. Natl. Acad. Sci. USA 83: 4143-4146 (1986) discloses inhibition of replication and expression of human immunodeficiency virus (HIV-1, then called HTLV-III) in cultured cells by synthetic oligonucleotide phosphodiesters complementary to viral RNA.

More recently, it has been reported that oligonucleotides having greater resistance to nucleolytic degradation than oligonucleotide phosphodiesters are more effective as antisense oligonucleotides. Agrawal, Tibtech 10: 152-158 (1992) has extensively reviewed the use of modified oligonucleotides as antiviral agents.

Sarin et al., Proc. Natl. Acad. Sci. USA 85: 7448-7451 (1988) teaches that oligodeoxynucleoside methylphosphonates are more active as inhibitors of HIV-1 than conventional oligodeoxynucleotides.

Agrawal et al., Proc. Natl. Acad. Sci. USA 85: 7079-7083 (1988) teaches that oligonucleotide phosphorothioates and various oligonucleotide phosphoramidates are more effective at inhibiting HIV-1 than conventional oligodeoxynucleotides.

Agrawal et al., Proc. Natl. Acad. Sci. USA 86: 7790-7794 (1989) discloses the advantage of oligonucleotide phosphorothioates in inhibiting HIV-1 in early and chronically infected cells.

An additional characteristic that renders oligonucleotides more effective as antisense agents is the ability to activate RNase H. Thus, oligonucleotide phosphorothioates, which are both resistant to nucleolytic degradation and activators of RNase H, are effective as inhibitors of HIV-1 and several other viruses.

Gao et al. Antimicrob. Agents and Chem. 34: 808 (1990) discloses inhibition of HSV by oligonucleotide phosphorothioates.

Storey et al., Nucleic Acids Res. 19: 4109 (1991) discloses inhibition of HPV by oligonucleotide phosphorothioates.

Leiter et al., Proc. Natl. Acad. Sci. USA 87: 3430 (1990) discloses inhibition of influenza virus by oligonucleotide phosphorothioates.

Unfortunately, oligonucleotide phosphorothioates increase resistance to nucleolytic degradation but do not provide complete resistance in vivo.

Agrawal et al., Proc. Natl. Acad. Sci. USA 88: 7595-7599 (1991) teaches that oligonucleotide phosphorothioates are extensively degraded from the 3' end in mice.

In addition, oligonucleotide phosphorothioates form less stable duplexes between the oligonucleotide and target than oligodeoxynucleotides phosphodiesters. To overcome these deficiencies, oligonucleotides having cap structures at the 3' terminus have been developed. Agrawal and Goodchild, Tetrahedron Lett. 28: 3539-3542 (1987) discloses the use of oligodeoxynucleoside methylphosphonates as 5' and 3' capping agents. Shaw et al., Nucleic Acids Res. 19: 747-750 (1991) discloses oligodeoxynucleotide phosphodiesters having blocking structures at the 3' end.

Temsamani et al., in Antisense Strategies, Annals of New York Academy of Sciences (in press) (1992) discloses 3' capped oligonucleotide phosphorothioates.

Even these nuclease resistant 3' capped oligonucleotides can become degraded eventually as the 3' capped end of these oligonucleotides is slowly digested by a combination of endonuclease and exonuclease activities.

There is, therefore, a need for oligonucleotides that form stable duplexes, resist nucleolytic degradation and activate RNase H, without the disadvantages of oligonucleotides that are known in the art. Ideally, such oligonucleotides should resist even the combined effect of endonucleases and exonucleases, should stably pair with target sequences at physiological temperatures, should activate Rnase H and should produce only nucleosides as degradation products.

Oligonucleotides having self-complementary structures that can result in hairpin formation are known in the art.

Germann et al., Biochemistry 24: 5698-5702 (1985) discloses a partially self-complementary 24-mer oligonucleotide, d(GC)₅ T₄(CG)₅, that undergoes a β-DNA to Z-DNA transition.

Hilbers et al., Biochimie 67: 685-695 (1985) discusses the dynamics of hairpin formation in a partially self-complementary oligonucleotide, dATCCTATₙTAGGAT.

Neither of these physical studies related to either oligonucleotide stability or to therapeutic use of oligonucleotides.

Thus, the prior art is devoid of any teaching or suggestion about using self-complementary oligonucleotides in the antisense oligonucleotide therapeutic approach, nor does it discuss the use of hairpin formation as a means of rendering an oligonucleotide resistant to nucleolytic degradation.

### BRIEF SUMMARY OF THE INVENTION

The invention relates to novel therapeutic agents used in the antisense oligonucleotide therapeutic approach. The invention provides improved antisense oligonucleotides that are resistant to nucleolytic degradation. Oligonucleotides according to the invention resist nucleolytic degradation, including the combined effect of endonucleases and exonucleases. Oligonucleotides according to the invention form stable hybrids with target sequences under physiological conditions, activate RNase H and produce only nucleosides as degradation products.

The advantages of oligonucleotides according to the invention, known as self-stabilized oligonucleotides, arise from the presence of two structural features: a target hybridizing region and a self-complementary region. The target hybridizing region comprises an oligonucleotide sequence that is complementary to a nucleic acid sequence that is from a plant or animal virus, a pathogenic organism, or a cellular gene or gene transcript, the abnormal gene expression or product of which results in a disease state. The self-complementary region comprises an oligonucleotide sequence that is complementary to a nucleic acid sequence within the oligonucleotide. Thus, at least when the oligonucleotide is not hybridized to a target nucleic acid sequence, the oligonucleotide forms a totally or partially double-stranded structure that is resistant to nucleolytic degradation. Since the inherent structure of these molecules confers resistance to nucleases, it is not necessary to use modified internucleoside linkages to confer such resistance, although of course, modified internucleoside linkages may be used. Thus, the use of oligonucleotide phosphodiesters or oligonucleotide phosphorothioates, both of which are degraded in vivo, is made feasible by oligonucleotides according to the invention. This results in oligonucleotides that activate RNase H, an important feature for the antisense therapeutic compound. Also, the use of oligonucleotide phosphodiesters provides more stable hybridization between therapeutic oligonucleotides and target sequences. Finally, degradation of such oligonucleotides results only in nucleotide breakdown products, thus minimizing potential toxicity. These advantages result in a superior therapeutic oligonucleotide.

The invention further provides self-stabilized ribozymes, since the self-complementary motif of the invention can be conveniently used with ribonucleotides. Such ribozymes according to the invention have generally typical ribozyme structure, except that they have a self-complementary region at or near the 5' or 3' end. This region confers nuclease resistance upon the ribozymes, making them more stable than ribozymes that are known in the art.

### BRIEF DESCRIPTION OF THE DRAWINGS

Figure 1 illustrates a self-stabilized oligonucleotide according to the invention in hairpin and hybridized configurations.

Figure 2 illustrates a self-stabilized oligonucleotide according to the invention in hammer-like configuration.

Figure 3 shows results of duplex stability studies for hybridization between oligonucleotides or self-stabilized oligonucleotides and complementary target oligonucleotides.

Figure 4 shows results of 3'-exonuclease treatment of oligonucleotides.

Figure 5 shows the structure of self-stabilized oligonucleotides used in Examples 1-4.

Figure 6 shows a mechanism of therapeutic action of self-stabilized oligonucleotides.

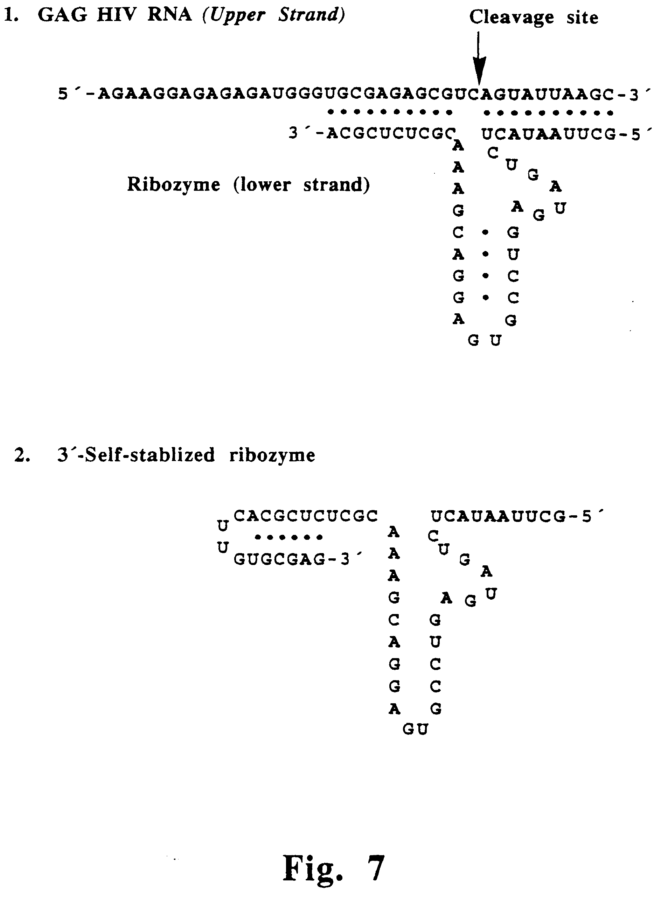

Figure 7 shows a self-stabilized ribozyme according to the invention. This example of a self-stabilized ribozyme according to the invention is complementary to the HIV gag region and results in the cleavage of a HIV gag mRNA.

### DETAILED DESCRIPTION OF THE PREFERRED EMBODIMENTS

The invention relates to novel therapeutic agents that are useful in the treatment of virus infections, infections by pathogenic organisms, and diseases arising from abnormal gene expression or gene products.

In a first aspect, the invention provides self-stabilized oligonucleotides that are more resistant to nucleolytic degradation than oligonucleotides that are known in the art. For purposes of the invention, the term oligonucleotide includes polymers of ribonucleotides, deoxyribonucleotides, or both, with ribonucleotide and/or deoxyribonucleotide monomers being connected together via 5' to 3' linkages which may include any of the linkages that are known in the antisense oligonucleotide art. In addition, the term oligonucleotides includes such molecules having modified nucleic acid bases and/or sugars, as well as such molecules having added substituents, such as diamines, cholesteryl or other lipophilic groups. Certain preferred combinations of monomers and inter-monomer linkages are discussed in greater detail below.

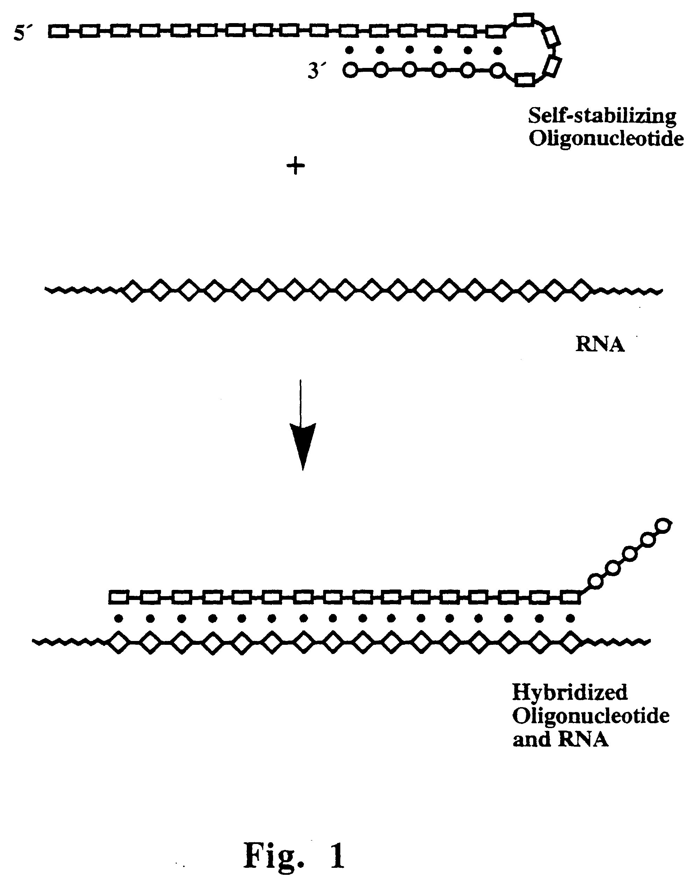

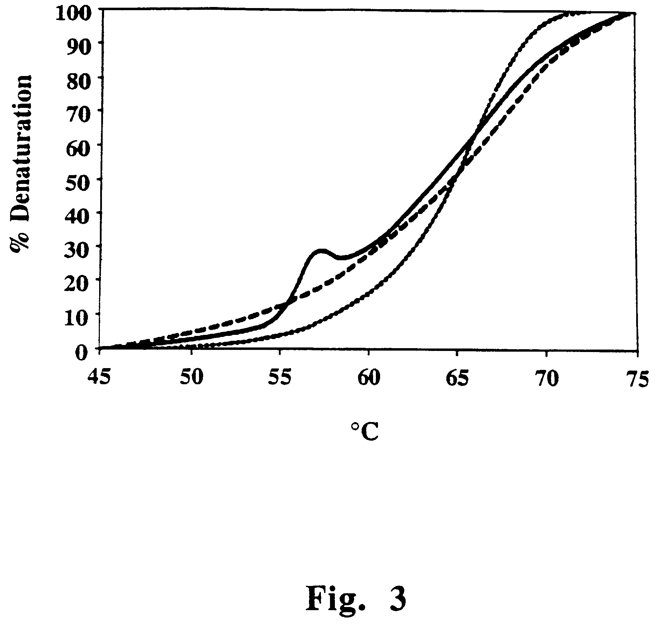

Oligonucleotides according to the invention are generally characterized by having two regions: a target hybridizing region and a self-complementary region. A first embodiment of a self-stabilized oligonucleotide according to the invention is shown in Figure 1. In this embodiment, the target hybridizing region is shown as connected rectangular squares, and the self-complementary region is shown as connected circles. The complementary nucleic acid sequence in a target RNA molecule is represented by connected diamonds. Hydrogen bonding between nucleotides is indicated by dots. The oligonucleotide is stabilized, i.e., rendered resistant to nucleolytic degradation from the 5' or 3' end by base-pairing between the target hybridizing region and the self-complementary regions and/or by base-pairing between complementary sequences within the self-complementary region. When the oligonucleotide encounters a nucleic acid molecule having a complementary nucleic acid sequence, base-pairing between the target hybridizing region and the self-complementary region of the oligonucleotide is disrupted and replaced by base-pairing between the target hybridizing region of the oligonucleotide and the complementary nucleic acid sequence of the target nucleic acid molecule. This disruption and replacement of base-pairing takes place because the intermolecular base-paired structure formed by the hybrid between the target nucleic acid sequence and the target hybridizing region is more thermodynamically stable than the intra-molecular base-paired structure formed by the self-complementary oligonucleotide. This phenomenon is illustrated in Figure 3 and discussed in greater detail in Example 4.

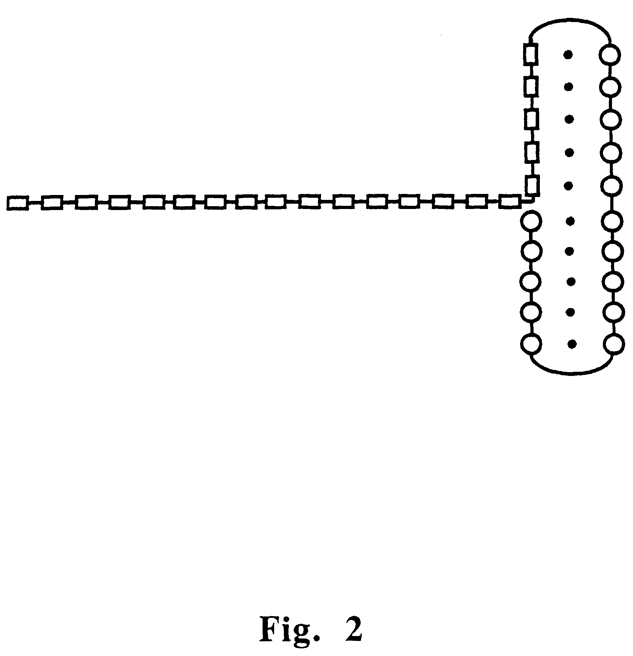

A second embodiment of an oligonucleotide according to the invention operates in a similar way as the first embodiment, but forms a different structure upon self-complementary base-pairing. This alternative embodiment forms a hammer-like structure as shown in Figure 2. In this embodiment, the self-complementary region contains oligonucleotide sequences that can base pair with other oligonucleotide sequences within the self-complementary region. The self complementary region may also contain oligonucleotide sequences that are complementary to the target hybridizing region.

The target hybridizing region of an oligonucleotide according to the invention has an oligonucleotide sequence that is complementary to a nucleic acid sequence that is from a virus, a pathogenic organism, or a cellular gene or gene transcript, the abnormal gene expression or product of which results in a disease state. Preferably the target hybridizing region is from about 8 to about 50 nucleotides in length. For purposes of the invention, the term "oligonucleotide sequence that is complementary to a nucleic acid sequence" is intended to mean an oligonucleotide sequence (2 to about 50 nucleotides) that hybridizes to the nucleic acid sequence under physiological conditions, e.g., by Watson-Crick base paring (interaction between oligonucleotide and single-stranded nucleic acid) or by Hoogsteen base pairing. (interaction between oligonucleotide and double-stranded nucleic acid) or by any other means. Suc. hybridization under physiological conditions is measured as a practical matter by observing interference with the function of the nucleic acid sequence.

The nucleic acid sequence to which the target hybridizing region of an oligonucleotide according to the invention is complementary will vary, depending upon the disease condition to be treated. In many cases the nucleic acid sequence will be a virus nucleic acid sequence. The use of antisense oligonucleotides to inhibit various viruses is well known, and has recently been reviewed in Agrawal, Tibtech 10:152-158 (1992). Viral nucleic acid sequences that are complementary to effective antisense oligonucleotides have been described for many viruses, including human immunodeficiency virus type 1 (U.S. Patent No. 4,806,463), Herpes simplex virus (U.S. Patent No. 4,689,320), Influenza virus (U.S. Patent No. 5, 194, 428), and Human papilloma virus (Storey et al., Nucleic Acids Res. 19:4109-4114 (1991)). Sequences complementary to any of these nucleic acid sequences can be used for the target hybridising region of oligonucleotides according to the invention, as can be oligonucleotide sequences complementary to nucleic acid sequences from any other virus. Additional viruses that have known nucleic acid sequences against which antisense oligonucleotides can be prepared include Foot and Mouth Disease Virus (See Robertson et al., J. Virology 54: 651 (1985); Harris et al., J. Virology 36: 659 (1980)), Yellow Fever Virus (See Rice et al., Science 229: 726 (1985)), Varicella-Zoster Virus (See Davison and Scott, J. Gen. Virology 67: 2279 (1986), and Cucumber Mosaic Virus (See Richards et al., Virology 89: 395 (1978)).

Alternatively, the target hybridizing region of oligonucleotides according to the invention can have an oligonucleotide sequence complementary to a nucleic acid sequence of a pathogenic organism. The nucleic acid sequences of many pathogenic organisms have been described, including the malaria organism, Plasmodium falciparum, and many pathogenic bacteria. Oligonucleotide sequences complementary to nucleic acid sequences from any such pathogenic organism can form the target hybridizing region of oligonucleotides according to the invention. Examples of pathogenic eukaryotes having known nucleic acid sequences against which antisense oligonucleotides can be prepared include Trypanosoma brucei gambiense and Leishmania (See Campbell et al., Nature 311: 350 (1984)), Fasciola hepatica (See Zurita et al., Proc. Natl. Acad. Sci. USA 84: 2340 (1987). Antifungal oligonucleotides can be prepared using a target hybridizing region having an oligonucleotide sequence that is complementary to a nucleic acid sequence from, e.g., the chitin synthetase gene, and antibacterial oligonucleotides can be prepared using, e.g., the alanine racemase gene.

In yet another embodiment, the target hybridizing region of oligonucleotides according to the invention can have an oligonucleotide sequence complementary to a cellular gene or gene transcript, the abnormal expression or product of which results in a disease state. The nucleic acid sequences of several such cellular genes have been described, including prion protein (Stahl and Prusiner, FASEB J. 5: 2799-2807 (1991)), the amyloid-like protein associated with Alzheimer's disease (U.S. Patent No. 5,015,570, the teachings of which are hereby incorporated by reference), and various well-known oncogenes and proto-oncogenes, such as c-myb, c-myc, c-abl, and n-ras. In addition, oligonucleotides that inhibit the synthesis of structural proteins or enzymes involved largely or exclusively in spermatogenesis, sperm motility, the binding of the sperm to the egg or any other step affecting sperm viability may be used as contraceptives for men. Similarly, contraceptives for women may be oligonucleotides that inhibit proteins or enzymes involved in ovulation, fertilization, implantation or in the biosynthesis of hormones involved in those processes.

Hypertension can be controlled by oligodeoxynucleotides that suppress the synthesis of angiotensin converting enzyme or related enzymes in the renin/angiotensin system; platelet aggregation can be controlled by suppression of the synthesis of enzymes necessary for the synthesis of thromboxane A2 for use in myocardial and cerebral circulatory disorders, infarcts, arteriosclerosis, embolism and thrombosis; deposition of cholesterol in arterial wall can be inhibited by suppression of the synthesis of fattyacyl co-enzyme A: cholesterol acyl transferase in arteriosclerosis; inhibition of the synthesis of cholinephosphotransferase may be useful in hypolipidemia.

There are numerous neural disorders in which hybridization arrest can be used to reduce or eliminate adverse effects of the disorder. For example, suppression of the synthesis of monoamine oxidase can be used in Parkinson's disease; suppression of catechol o-methyl transferase can be used to treat depression; and suppression of indole N-methyl transferase can be used in treating schizophrenia.

Suppression of selected enzymes in the arachidonic acid cascade which leads to prostaglandins and leukotrienes may be useful in the control of platelet aggregation, allergy, inflammations, pain and asthma.

Suppression of the protein expressed by the multidrug resistance (mdr) gene, which is responsible for development of resistance to a variety of anti-cancer drugs and is a major impediment in chemotherapy may prove to be beneficial in the treatment of cancer. Oligonucleotide sequences complementary to nucleic acid sequences from any of these genes can be used for the target hybridizing region of oligonucleotides according to the invention, as can be oligonucleotide sequences complementary to any other cellular gene or gene transcript, the abnormal expression or product of which results in a disease state.

Antisense regulation of gene expression in plant cells has been described in U.S. Patent No. 5,107,065, the teachings of which are hereby incorporated by reference.

In a second aspect, the invention provides nuclease resistant oligonucleotides that activate RNase H. The target hybridizing region of oligonucleotides according to the invention may contain ribonucleotides, deoxyribonucleotides or any analogs of ribonucleotides or deoxyribonucleotides. In one preferred embodiment, this region is composed of ribonucleotides. In another preferred embodiment, this region is composed of deoxyribonucleotides. In yet another preferred embodiment, this region comprises a mixture of ribonucleotides and deoxyribonucleotides. An additional preferred embodiment has a target hybridizing region comprising oligonucleotide phosphodiesters, phosphorothioates, or phosphorodithicates, or mixtures of these with ribonucleotides or deoxyribonucleotides. These preferred embodiments all provide for the activation of RNase H, as long as four or more contiguous deoxyribonucleotide phosphodiesters, phosphorothioates, or phosphorodithioates are present. Of course, other embodiments employing target hybridizing regions that do not activate RNase H can also be made.

Synthesis procedures for each of these embodiments are well known in the art. Both oligodeoxyribonucleotide phosphodiesters and oligodeoxyribonucleotide phosphorothioates and their analogs can be synthesized by the H-phosphonate approach described in U.S. Patent No. 5,149 , 798,

the teachings of which are hereby incorporated by reference. The H-phosphonate approach can also be used to synthesize oligoribonucleotides and oligoribonucleotide analogs, as described in Agrawal and Tang, Tetrahedron Lett. 31 : 7541-7544 (1990). Synthesis of oligonucleotide phosphorodithioates is also known in the art.

Of course, many other embodiments are possible, and those skilled in the art will recognize that other analogs or combinations of analogs can be used in the target hybridizing region of oligonucleotides according to the invention. Such analogs are characterized by having internucleoside linkages other than the natural phosphodiester linkage. The synthesis of many such analogs is well known in the art, including analogs having alkylphosphonate, (Agrawal and Goodchild, Tetrahedron Lett. 28: 3539-3542 (1987)) or phosphoramidate (Agrawal et al., Proc. Natl. Acad. Sci. USA 85: 7079-7083 (1988)) linkages.

The second significant region of self-stabilized oligonucleotides according to the invention is the self-complementary region. The self-complementary region contains an oligonucleotide sequence that is complementary to a nucleic acid sequence within the oligonucleotide. This oligonucleotide sequence and this complementary nucleic acid sequence may be within the target hybridizing region or within the self-complementary region, or they may span both regions. The complementary sequences form base pairs, resulting in the formation of a hairpin structure, as shown in Figure 1, or a hammer-like structure, as shown in Figure 2. Either the hairpin structure or the hammer-like structure can have loops resulting from non-base-paired nucleotides, as shown in Figure 1 for the hairpin structure, or can be devoid of such loops, as shown in Figure 2 for the hammer-like structure. The number of base-pairs to be formed by intra-molecular hybridization involving the self-complementary region may vary, but should be adequate to maintain a double-stranded structure so that the 3' end is not accessible to exonucleases. Generally, about 4 or more base-pairs will be necessary to maintain such a double-stranded structure. In a preferred embodiment, there are about 10 intramolecular base-pairs formed in the self-stabilized oligonucleotide, with the 10 base pairs being consecutive and involving the 3'-most nucleotides. Of course, the intra-molecular base-pairing can be so extensive as to involve every nucleotide of the oligonucleotide. Preferably, this will involve a self-complementary region of about 50 nucleotides or less.

In one preferred embodiment the self-complementary region may be connected to the target hybridizing region by a suitable non-nucleic acid linker. Examples of such linkers include substituted or unsubstituted alkyl groups. In one most preferred embodiment the linker is a (ethylene glycol)₁₋₆ linker. At the larger size for this linker, the synthesis may be conveniently carried out by using commercially available triethylene glycol that has a dimethyltrityl protective group at one end and a cyanoethylphosphoramidite group at the other end.

The self-complementary region may contain ribonucleotides, deoxyribonucleotides, analogs of ribonucleotides or deoxyribonucleotides having artificial linkages, or combinations of any of the above. The ability to activate RNase H is not important for the self-complementary region, so nucleotides having artificial linkages that do not activate RNase H can be used in this region without diminishing the effectiveness of the oligonucleotide. Thus, in addition to phosphodiester and phosphorothioate or phosphorodithioate linkages, this region may also or alternatively contain phosphoramidate (including N-substituted phosphoramidates) , alkylphosphonate, alkylphosphonothioate linkages as well as non-phosphate containing linkages, such as sulfone, sulfate, and keto linkages. Of course, in non-RNase H activating embodiments of self-stabilized oligonucleotides according to the invention, any of these linkages can be used in the target hybridizing region as well.

In one preferred embodiment, the self-stabilized oligonucleotide is rendered hyperstabilized. This may be accomplished by incorporating into the self-complementary region one or more ribonucleotides or 2'-0-Me-ribonucleotides, wherein the complementary portion of the target hybridizing region is DNA. Alternatively, the complementary region of the target hybridizing region may contain ribonucleotides or 2'-O-Me-ribonucleotides, and the self-complementary region may contain DNA. These oligonucleotides will be hyperstabilized because the interaction between DNA and RNA is more stable than the interaction between DNA and DNA. Yet another way in which the self-complementary region (and/or the linked region) may be modified to yield a hyperstabilized self-stabilized oligonucleotide is to incorporate one or more intercalating agent molecule. These oligonucleotides are hyperstabilized because the intercalating agent stabilizes the hybrid formed between the self-complementary region and the target hybridizing region. Any intercalating agent is acceptable for this purpose. Preferred intercalating agents include acridine and ethidium. oligonucleotides containing acridine are readily prepared by using the commercially available acridine-ON phosphoramidite, or 3'-acridine-ON CPG (Clontech Laboratories, Inc.).

In a third aspect, the invention provides ribozymes that are more stable than ribozymes that are known in the art. Ribozymes are catalytic RNA molecules that cleave internucleoside bonds. The stability of such ribozymes according to the invention is provided by the incorporation of a self-complementary region at or near the 5' or 3' end of the ribozyme molecule. This self-complementary region results in the formation of a hairpin or hammer-like structure, thus rendering the 5' or 3' end of the molecule double-stranded, which causes the ribozyme molecule to resist nucleolytic degradation. The structure and function of ribozymes is generally taught in U.S. Patent No. 4,987,071.

In a fourth aspect, the invention provides an in vitro method for inhibiting the gene expression of a virus, a pathogenic organism or a cellular gene, the method comprising the step of contacting self-stabilized oligonucleotides or ribozymes according to the invention will cells infected with the virus or pathogenic organism in the former two cases or to cells generally in the latter case.

In a fifth aspect, the invention relates to the use of a self stabilized oligonucleotide as defined above for the preparation of a pharmaceutical composition for inhibiting the gene expression of a virus, a pathogenic organism, or a cellular gene. The pharmaceutical composition comprises a pharmaceutically acceptable carrier. Preferably, the routes of administration will include oral, intranasal, rectal and topical administration. In such methods of treatment the self-stabilized oligonucleotides may be administered in conjunction with other therapeutic agents, e.g., AZT in the case of AIDS.

A variety of viral diseases may be treated including AIDS, ARC, oral or genital herpes, papilloma warts, flu, foot and mouth disease, yellow fever, chicken pox, shingles, HTLV-leukemia, and hepatitis. Among fungal diseases treatable are candidiasis, histoplasmosis, cryptococcosis, blastomycosis, aspergillosis, sporotrichosis, chromomycosis, dermatophytosis and coccidioidomycosis. The method can also be used to treat rickettsial diseases (e.g., typhus, Rocky Mountain spotted fever) , as well as sexually transmitted diseases caused by Chlamydia trachomatis or lymphogranuloma venereum. A variety of parasitic diseases can be treated, including amebiasis, Chegas, disease, toxoplasmosis, pneumocystosis, giardiasis, cryptosporidiosis, trichomoniasis, and Pneumocystis carini pneumonia; also worm (helminthic) diseases such as ascariasis filariasis, trichinosis, schistosomiasis and nematode or cestode infections. Malaria can be treated regardless of whether it is caused by P. falciparum, P. vivax, P. orale, or P. malariae.

The infectious diseases identified above can all be treated because the infectious agents for these diseases are known and thus self-stabilized oligonucleotides according to the invention can be prepared, having a target hybridizing region that has an oligonucleotide sequence that is complementary to a nucleic acid sequence that is an essential nucleic acid sequence for the propagation of the infectious agent, such as an essential gene.

Other disease states or conditions that are treatable result from an abnormal expression or product of a cellular gene. These conditions can be treated by administration of self-stabilized oligonucleotides according to the invention, and have been discussed earlier in this disclosure.

The invention provides numerous advantages over oligonucleotides that are known in the art. First, the self-stabilized oligonucleotides according to the invention have a longer half-life than most known oligonucleotides, thereby lowering the dosage that will be required for therapeutic efficacy. Even greater resistance to nuclease degradation can be provided by using nuclease resistant internucleoside linkages near or cap structures at one or both ends of the oligonucleotide. Second, the enzymatic stability afforded by the base-paired structures involving the self-complementary sequences allows the use of oligonucleotide phosphodiesters, which otherwise are rapidly degraded. This provides the advantages of increased duplex stability and RNase H activation, which are not both provided by any nuclease resistant oligonucleotide known in the art. The advantage of RNase H activation is retained when oligonucleotide phosphorothioates or phosphorodithioates are used. A third advantage is that the only degradation product of several embodiments of oligonucleotides according to the invention is nucleotides, e.g., nucleoside monophosphates and/or nucleoside monothiophosphates. Finally, the invention allows the use of either deoxyribonucleosides or ribonucleosides. The ability to use the latter makes the invention readily adaptable for use with ribozymes, for which enzymatic stability is critical.

The following examples are provided to further illustrate certain aspects of preferred embodiments of the invention, and are not intended to be limiting in nature.

### EXAMPLE 1

### Nuclease Resistance of Oligonucleotide Phosphodiesters

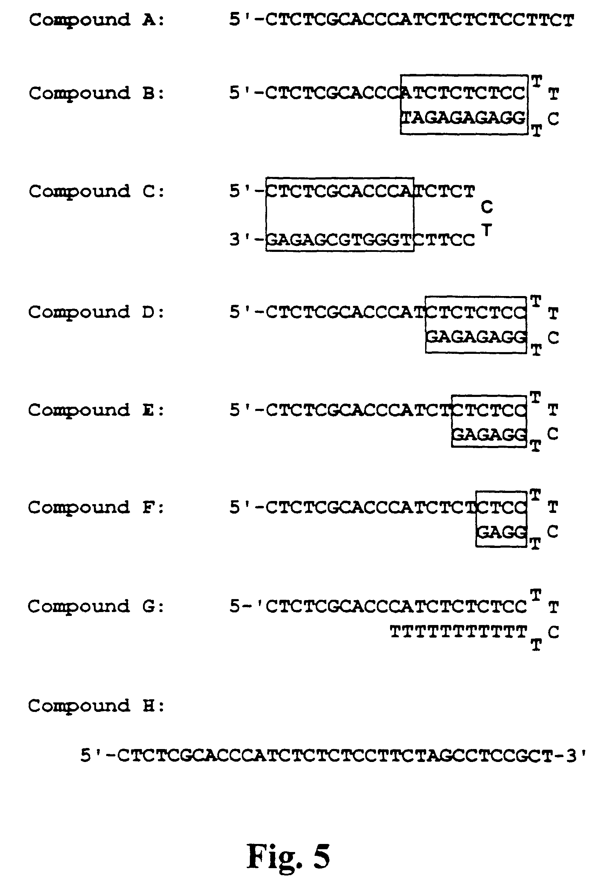

The oligonucleotides used in the study are shown in Figure 5. Oligonucleotide CMPD A is complementary to a portion of the gag region of HIV-1. Oligonucleotide CMPD B uses this same region as a target hybridizing region, but adds a 3' self-complementary region of 10 nucleotides. Oligonucleotides CMPD E and CMPD F are identical to CMPD B, except that the self-complementary regions of CMPD E and CMPD F are 6 and 4 nucleotides, respectively. Oligonucleotide CMPD G is identical to CMPD A, except that it has 10 mismatched nucleotides (T₁₀) added at its 3' end.

The oligonucleotides were tested for their relative resistance to 3' exonucleolytic degradation. For each oligonucleotide, 0.4 A₂₆₀ units of oligonucleotide was lyophilized, dissolved in 0.5ml buffer (10mM Tris, 10mM MgCl₂, pH 8.5) and mixed with 5µl (1.5 milliunits) of snake venom phosphodiesterase (SVPD). The mixture was incubated at 37°C in a thermally regulated cell and A₂₆₀ was plotted against time. Oligonucleotide degradation was measured as function of increase in hyperchromicity.

The results of these experiments are shown in Table I, below. These results demonstrate that self-stabilized oligonucleotide phosphodiesters according to the invention are far more resistant to 3' exoncleolytic degradation than either oligonucleotide phosphodiesters or oligonucleotide phosphodiesters having a non-complementary tail.

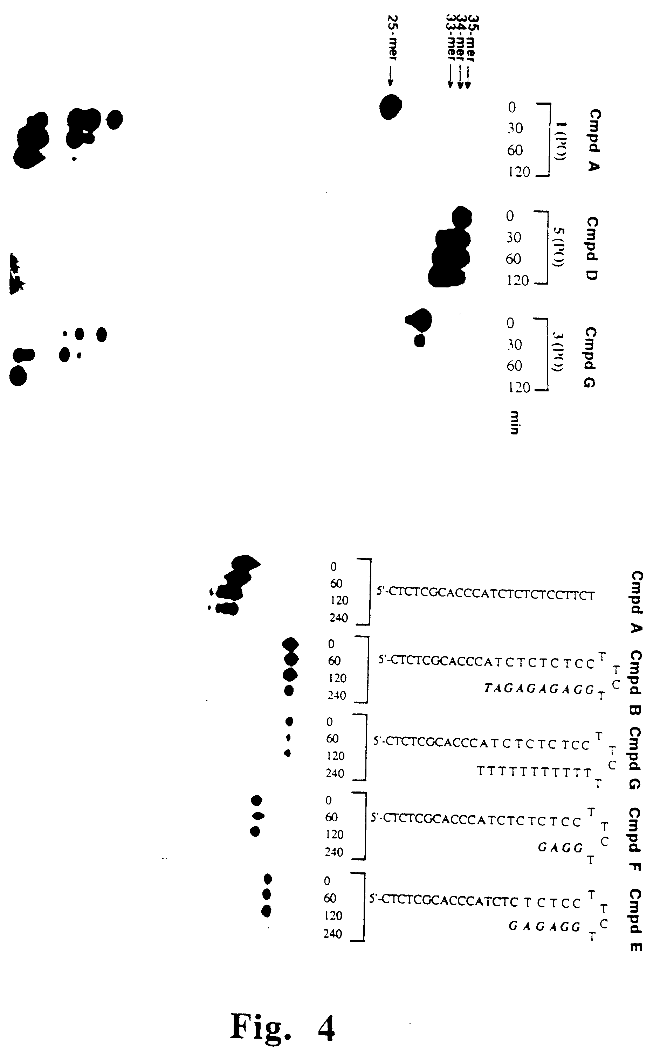

In addition to the testing described above, the oligonucleotides were also subjected to DNA Polymerase I 3'-exonuclease digestion. As shown in Figure 4 the non-self-stabilized oligonucleotides, CMPDs A and G were digested to completion in 30 minutes, whereas self-stabilized CMPD B was only partly digested over 30 minutes.

**TABLE I**

| **HALF-LIFE OF OLIGONUCLEOTIDES** | |
|---|---|
| Oligonucleotide | t½ for SVPD digestion |
| CMPD A | 75 seconds |
| CMPD G | 75 seconds |
| CMPD B | 950 seconds |

### EXAMPLE 2

### Nuclease Resistance of Oligonucleotide Phosphorothioates

To test the relative nuclease resistance of self-stabilized and non-self-stabilized oligonucleotide phosphorothioates, a DNA Polymerase I 3'- exonuclease activity assay was used, because of the slow degradation of oligonucleotide phosphorothioates by SVPD.

All oligonucleotides were labelled at the 5'- end with gamma-³²p-ATP and kinase. To a solution of 40 pmole 5'-labelled oligonucleotide in 20µl buffer (40 mM Tris. HCl pH 8.0, 10 mM MgCl₂, 5 mM DTT, 50 mM KCl, 50µg/ml BSA), 5 units DNA polymerase I was added and incubated at 37°C. Aliquots of 4µl were taken at 0, 30, 60, 120 minutes and were mixed with 6 µl stop solution (98% formamide, 10 mM EDTA, 0.1% xylene cyanol, 0.1% bromophenol blue). The samples were analyzed by 15% acrylamide gel (urea) and autoradiography.

The results are shown in Figure 4. Phosphorothioate analog of CMPD A was digested to almost 50% within 4 hours. The phosphorothioate analog of CMPD B, however, was undegraded after 4 hours. Phosphorothioate analogs of CMPD E and F, which have 6 and 4 base pairs of self-complementary sequence, respectively were also found to be stable. Phosphorothioate analog of CMPD G, having extended structure, but no self-complementary region, was digested at same rate as CMPD A. These results demonstrate that self-stabilized oligonucleotide phosphorothioates are far more resistant to nucleolytic degradation than are non-self-stabilized oligonucleotide phosphorothioates.

### EXAMPLE 3

### Anti-HIV Activity Of Oligonucleotides

Self-stabilized and non-self stabilized oligonucleotide phosphodiesters were tested for their ability to inhibit HIV-1 in tissue culture. The oligonucleotides used in this study are shown in Figure 5.

H9 lymphocytes were infected with HIV-1 virions (≡0.01 - 0.1 TCID₅₀/cell) for one hour at 37°C. After one hour, unadsorbed virions were washed and the infected cells were divided among wells of 24 well plates. To the infected cells, an appropriate concentration (from stock solution) of oligonucleotide was added to obtain the required concentration in 2 ml medium. In a positive control experiment ddC or AZT was added. The cells were then cultured for three days. At the end of three days, supernatant from the infected culture was collected and measured for p24 expression by ELISA. The level of expression of p24 was compared between oligonucleotide treated and untreated (no drug) infected cells.

Cytotoxicity of oligonucleotides was studied by culturing the cells with increasing concentration of oligonucleotide and by the trypan blue dye exclusion method.

The results of two experiments are shown in Table III, below.

**TABLE III**

| Anti-HIV Activity of Oligonucleotides | | | | |
|---|---|---|---|---|
| Experiment 1 | | | | |
| | Concentration ( g/ml) | Inhibition of p24 (%) | % Cell Survival | IC₅₀ (g/ml) . |
| CMPD A | 25 | 90 | 93 | 2 |
| | 5 | 89 | 103 | |
| | 1 | 15 | 94 | |
| | 0.2 | 26 | 97 | |
| CMPD B | 25 | 90 | 95 | 0.25 |
| | 5 | 85 | 92 | |
| | 1 | 84 | 94 | |
| | 0.2 | 46 | 103 | |
| CMPD G | 25 | 86 | 106 | 0.5 |
| | 5 | 86 | 105 | |
| | 1 | 81 | 106 | |
| | 0.2 | 0 | 109 | |
| AZT | 0.2 | 90 | 95 | 0.037µM |
| | 0.04 | 73 | 98 | |
| | 0.08 | 44 | 104 | |
| | .0016 | 6 | 108 | |

| Experiment 2 | | | | |
|---|---|---|---|---|
| | **Concentration ( g/ml)** | **Inhibition of p24 (%)** | **% Cell Survival** | **IC**_{**50**} **( g/ml)** |
| **CMPD A** | 5 | 66 | 93 | 2.8 |
| | 1 | 20 | 101 | |
| | 0.2 | 21 | 107 | |
| | 0.04 | 0 | 102 | |
| **CMPD B** | 5 | 93 | 89 | 0.35 |
| | 1 | 81 | 99 | |
| | 0.2 | 33 | 103 | |
| | 0.04 | 0 | 104 | |
| **CMPD E** | 5 | 89 | 93 | 0.45 |
| | 1 | 41 | 100 | |
| | 0.2 | 19 | 99 | |
| | 0.04 | 0 | 102 | |
| **CMPD F** | 5 | 89 | 93 | 1.5 |
| | 1 | 41 | 100 | |
| | 0.2 | 19 | 99 | |
| | 0.04 | 0 | 102 | |
| **AZT** | 0.2 | 89 | 93 | 0.1µm |
| | 0.04 | 65 | 98 | |
| | 0.008 | 5 | 101 | |
| | 0.0016 | 6 | 103 | |

All self-stabilized oligonucleotides exhibited greater anti-HIV activity than CMPD A, the non-self-stabilized oligonucleotide. Greatest activity was observed for the self-stabilized oligonucleotide having 10 self-complementary nucleotides, which exhibited nearly ten times the activity of the oligonucleotide phosphodiester. The oligonucleotide CMPD G, which has a poly T tail, also showed some increase in activity, probably as a result of stabilization from hybridization with polyA from mRNA in the H9 cells.

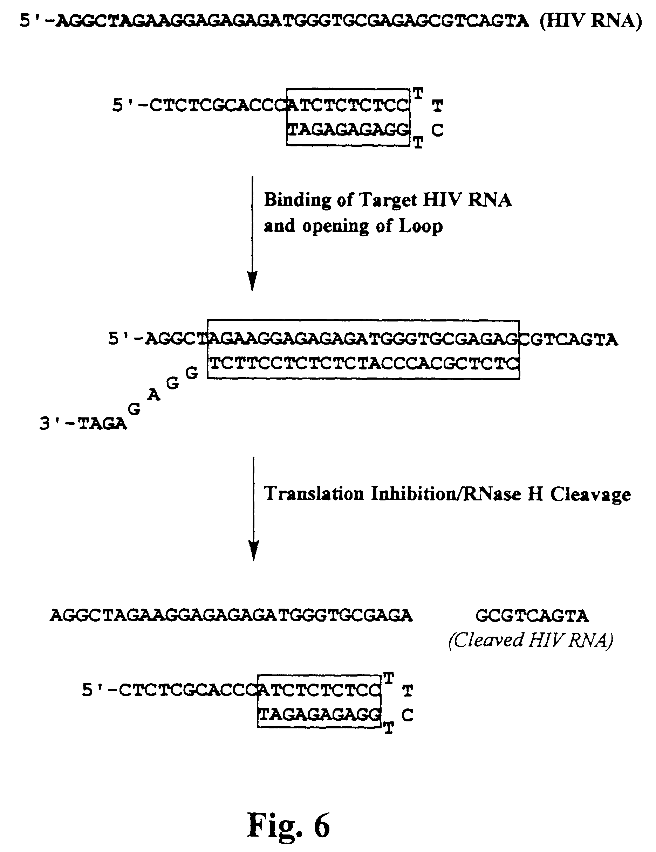

The probable mechanism of action of the CMPD B oligonucleotide is shown in Figure 6. The oligonucleotide enters the cell in a partially double-stranded form as a result of intramolecular base-paring involving the self-complementary region. As the oligonucleotide encounters an HIV RNA molecule having a nucleic acid sequence that is complementary to the oligonucleotide sequence of the target hybridizing region of the oligonucleotide, hybridization occurs between the HIV RNA and the target hybridizing region. This hybridization disrupts the intramolecular hybridization involving the self-complementary region. RNase H activity then cleaves the HIV RNA, allowing the oligonucleotide to once again self-stabilize by intramolecular base-pairing.

To test the relative anti-HIV activity of additional oligonucleotide structures, the above experiment was repeated using additional oligonucleotides, as well as the oligonucleotides described in Experiments 1 & 2. The additional oligonucleotides are shown in Figure 5. These additional oligonucleotides were CMPD C, in which the self-complementary region is complementary to the oligonucleotide through its 5' end; CMPD D, which has a 8 nucleotide self-complementary region; and CMPD H, a 35 mer oligonucleotide having perfect complementarity to the HIV gag RNA, but no self-conplementary region. The results of this third experiment are shown in Table IV, below.

These results demonstrate that fully self-complementary self-stabilized oligonucleotides are roughly equivalent in anti-HIV activity to partially self-complementary self-stabilized oligonuoleotides. The results also show that four self-complementary nucleotides are adequate to confer enhanced efficacy.

**TABLE IV**

| Anti-HIV Activity of Oligonucleotides | | | | |
|---|---|---|---|---|
| Experiment 3 | | | | |
| | **Concentration (g/ml)** | **Inbibition of p24 (%)** | **% Cell Survival** | **IC**_{**50**} **( g/ml)** |
| **CMPD A** | 5.0 | 92 | 97 | 1.7 |
| | 1.0 | 36 | 103 | |
| | 0.2 | 23 | 102 | |
| | 0.04 | 0 | 109 | |
| **CMPD B** | 5.0 | 95 (97)* | 98 (97)* | 0.5 (0.2)* |
| | 1.0 | 61 (74)* | 101 (102)* | |
| | 0.2 | 33 (49)* | 104 (103)* | |
| | 0.04 | 0 (19)* | 11 (106)* | |
| **CMPD G** | 5.0 | 94 | 97 | 0.6 |
| | 1.0 | 68 | 104 | |
| | 0.2 | 11 | 109 | |
| | 0.04 | 12 | 110 | |
| **CMPD E** | 5.0 | 92 | 98 | 0.8 |
| | 1.0 | 55 | 101 | |
| | 0.2 | 13 | 103 | |
| | 0.04 | 0 | 107 | |
| **CMPD F** | 5.0 | 95 | 99 | 0.25 |
| | 1.0 | 64 | 102 | |
| | 0.2 | 48 | 104 | |
| | 0.04 | 22 | 109 | |
| **CMPD C** | 5.0 | 94 | 96 | 0.3 |
| | 1.0 | 76 | 101 | |
| | 0.2 | 39 | 103 | |
| | 0.04 | 17 | 106 | |

| | Concentration (µ/ml) | Inbibition of p24 (%) | % Cell Survival | IC₅₀ ( g/ml) |
|---|---|---|---|---|
| **CMPD H** | 5 | 92 | 93 | 0.26 |
| | 1 | 88 | 101 | |
| | .2 | 43 | 98 | |
| | .04 | 0 | 102 | |
| | | | | |
| **CMPD D** | 5 | 80 | 93 | 0.4 |
| | 1 | 76 | 100 | |
| | .2 | 30 | 108 | |
| | .04 | 3 | 109 | |

| | | | | |
|---|---|---|---|---|
| * Results of second independent screening. | | | | |

### EXAMPLE 4

### Stability of Duplexes Between Self-Stabilized Oligonucleotides and Complementary Oligonucleotides

To test the stability of duplexes formed between self-stabilized oligonucleotides and complementary nucleic acid sequences hybridization studies were carried out. In a first study oligonucleotide CMPD A, which lacks self-complementary sequences, was mixed at room temperature with a complementary 25-mer oligonucleotide. The mixture was then gradually heated and increase in hyperchromicity was plotted against increase in temperature. In this study, the results of which are shown as a dotted line in Figure 3, the melting temperature of the duplex was found to be about 65°C.

In a second study CMPD B, having the same target hybridizing region as CMPD A and a 10 nucleotide self-complementary region, was mixed with the same 25-mer oligonucleotide at room temperature. The mixture was then gradually heated and increase in hyperchromicity was plotted against increase in temperature. The results are shown as a solid line in Figure 3. This time, in addition to the melting observed at about 65°C, an earlier increase in hyperchromicity was observed at about 58°C, corresponding to the melting of the intra molecular hydrogen bonds of the hairpin structure. This result indicates that the intramolecular base pairing involving the self-complementary region is less thermodynamically stable than the intermolecular base pairing between the target hybridizing region and a complementary oligonucleotide.

To further test the increased stability of the intermolecular base pairing relative to the intramolecular base pairing, CMPD B was then mixed with the same complementary 25-mer oligonucleotide and heated to 80°C, then allowed to cool to room temperature. This mixture was then gradually heated and increase in hyperchromicity was plotted against increase in temperature. The results are shown as a dashed line in Figure 3. Only a single melting temperature of about 65°C was observed, indicating that the intermolecular base pairing between CMPD B and the complementary 25-mer oligonucleotide is favored in competition with intramolecular base pairing involving the self-complementary region.

These results demonstrate that self-stabilized oligonucleotides will hybridize to complementary nucleic acid sequences withstanding the presence of oligonucleotide sequences within the oligonucleotide that are complementary to the target hybridizing region. Since it is well known that certain types of oligonucleotide structures hybridize more stably than certain other types of oligonucleotide structures (e.g., RNA:DNA hybrids >DNA:DNA hybrids and phosphodiester - containing oligonucleotides > phosphorothioate methylphosphonate or phosphoramidate - containing oligonucleotides), these results also indicate that the preferential target hybridizing effect may be enhanced by designing the self-stabilized oligonucleotide such that the hybridization between the target hybridizing region and the target sequence involves more stably pairing oligonucleotide structures than the hybridization involving the self-complementary region.

Those skilled in the art will recognize that self-complementary regions can be prepared according to the above teachings and combined with a wide variety of target hybridizing regions.

### EXAMPLE 5

### Hyperstabilized Self-Stabilized Oligonucleotides

To provide oligonucleotides having a more stable interaction between the self-complementary region and the target hybridizing region, oligodeoxynucleoside phosphodiesters or oligodeoxynucleoside phosphorothioates were prepared that had 2-O-Me-ribonucleosides in the self-complementary region. As shown in Table V below, such oligonucleotides had a hyperstabilized interaction between the self-complementary region and the target hybridizing region. Nevertheless, these oligonucleotides continued to favor formation of intermolecular hybrids with complementary DNA, relative to molecules containing intramolecular hybrids.

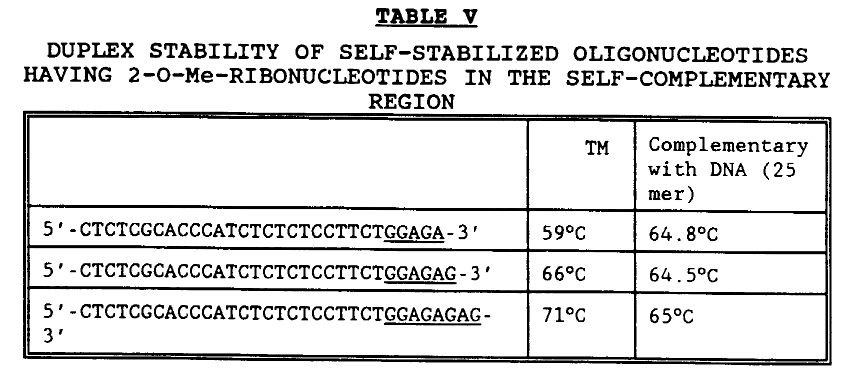

Another class of hyperstabilized self-stabilized oligonucleotides was prepared by covalently linking an acridine molecule to the terminus of the self-complementary region. These molecules also demonstrated hyperstability of the interaction between the target hybridizing region and the self-complementary region. Nevertheless, these molecules still preferentially formed intermolecular hybrids with complementary DNA, relative to forming intramolecular hybrids.

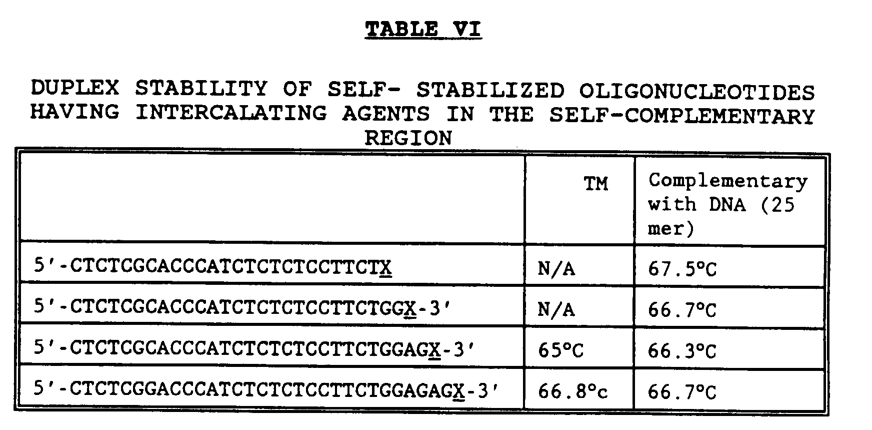

These results indicate that it is possible to construct hyperstabilized self-stabilized oligonucleotides having very stable interactions between the self-complementary region and the target hybridizing region, without interfering with the ability of the oligonucleotide to form intermolecular hybrids with a target nucleic acid.

## Claims

1. A self-stabilized oligonucleotide comprising a target hybridizing region and a self-complementary region,
wherein the target hybridizing region comprises an oligonucleotide sequence of at least 8 nucleotides which is complementary to a nucleic acid sequence that is from a virus, a pathogenic organism or a cellular gene,
wherein the self-complementary region comprises an oligonucleotide sequence of at least 4 nucleotides which is complementary to a nucleic acid sequence that is within the self-stabilized oligonucleotide,
wherein either the oligonucleotide sequence of the self-complementary region or the complementary nucleic acid sequence within the self-stabilized oligonucleotide comprises the 3' most sequence of nucleotides in the self-stabilized oligonucleotide,
wherein the oligonucleotide sequence of the self-complementary region and the complementary nucleic acid sequence within the self-stabilized oligonucleotide form a stable duplex under physiological conditions,
provided that no portion of the target hybridizing region is between the oligonucleotide sequence of the self-complementary region and the complementary nucleic acid sequence within the self-stabilized oligonucleotide,
further provided that the target hybridizing region does not comprise the entire molecule and
further provided that the target hybridizing region and the complementary nucleic acid sequence of the self-complementary region within the self-stabilized oligonucleotide have at least one nucleotide in common.

2. A self-stabilized oligonucleotide according to claim 1, wherein the target hybridizing region includes four or more contiguous deoxyribonucleotide phosphodiesters, phosphorothioates, or phosphorodithioates.

3. A self-stabilized oligonucleotide according to claim 1 or 2, wherein the self-complementary region comprises nucleotides selected from the group consisting of: deoxyribonucleotide or ribonucleotide phosphodiesters, phosphotriesters, phosphorothioates, phosphorodithioates, phosphoramidates, alkylphosphonates, and alkylphosphonothioates.

4. A self-stabilized oligonucleotide according to anyone of claims 1 to 3, wherein the virus is selected from the group consisting of: human immunodeficiency virus, herpes simplex virus, human papilloma virus, influenza virus, foot and mouth disease virus, yellow fever virus, Varicella-Zoster virus, and cucumber mosaic virus,
wherein the pathogenic organism is selected from the group consisting of *Plasmodium falciparum, Trypanosoma brucei, Leishmania*, and *Fasciola hepatica*, and
wherein the cellular gene is selected from a gene encoding a prion protein or Alzheimer's amyloid-like protein or from oncogenes or proto-oncogenes.

5. The self-stabilized oligonucleotide according to anyone of claims 1 to 4, wherein the target hybridizing region comprises said oligonucleotide sequence from about 8 to about 50 nucleotides and the self-complementary region comprises said oligonucleotide sequence of 6, 8, or about 10 nucleotides.

6. The self-stabilized oligonucleotide according to claim 5, wherein said self-complementary region comprises said oligonucleotide sequence of about 10 nucleotides.

7. An in vitro method of inhibiting the gene expression of a virus, a pathogenic organism, or a cellular gene, comprising providing a self-stabilized oligonucleotide to virus or pathogen infected cells, or to uninfected cells, respectively, the self-stabilized oligonucleotide comprising a target hybridizing region and a self-complementary region,
wherein the target hybridizing region comprises an oligonucleotide sequence of at least 8 nucleotides which is complementary to a nucleic acid sequence that is from a virus, a pathogenic organism or a cellular gene,
wherein the self-complementary region comprises an oligonucleotide sequence of at least 4 nucleotides which is complementary to a nucleic acid sequence that is within the self-stabilized oligonucleotide,
wherein either the oligonucleotide sequence of the self-complementary region or the complementary nucleic acid sequence within the self-stabilized oligonucleotide comprises the 3' most sequence of nucleotides in the self-stabilized oligonucleotide,
wherein the oligonucleotide sequence of the self-complementary region and the complementary nucleic acid sequence within the self-stabilized oligonucleotide form a stable duplex under physiological conditions,
provided that no portion of the target hybridizing region is between the oligonucleotide sequence of the self-complementary region and the complementary nucleic acid sequence within the self-stabilized oligonucleotide,
further provided that the target hybridizing region does not comprise the entire molecule and
further provided that the target hybridizing region and the complementary nucleic acid sequence of the self-complementary region within the self-stabilized oligonucleotide have at least one nucleotide in common.

8. The method according to claim 7, wherein the self-complementary region comprises said oligonucleotide sequence of about 10 nucleotides which is complementary to a nucleic acid sequence that is within the self-stabilized oligonucleotide.

9. The method according to claim 7 or 8, wherein the target hybridizing region includes 4 or more contiguous deoxyribonucleotide phosphodiesters, phosphorothioates, or phosphorodithioates.

10. The method according to anyone of claims 7 to 9, wherein the virus is selected from the group consisting or human immunodeficiency virus, herpes simplex virus, human papillomavirus, influenza virus, foot and mouth disease virus, yellow fever virus, Varicella-Zoster virus and cucumber mosaic virus,
wherein the pathogenic organism is selected from the group consisting of *Plasmodium falciparum, Trypanosoma brucei, Leishmanic,* and *Fasciola hepatica*, and
wherein the cellular gene is selected from a gene encoding a prion protein or Alzheimer's amyloid-like protein or from oncogenes or proto-oncogenes.

11. The use of a self-stabilized oligonucleotide according to anyone of claims 1 to 6 for the preparation of a pharmaceutical composition for inhibiting the gene expression of a virus, a pathogenic organism, or a cellular gene.

## Patentansprüche

1. Selbst-stabilisiertes Oligonukleotid umfassend eine Ziel-hybridisierende Region und eine selbst-komplementäre Region,
wobei die Ziel-hybridisierende Region eine Oligonukleotidsequenz von mindestens 8 Nukleotiden komplementär zu einer Nukleinsäuresequenz eines Virus, eines pathogenen Organismus oder eines zellulären Gens umfaßt,
wobei die selbst-komplementäre Region eine Oligonukleotidsequenz von mindestens vier Nukleotiden komplementär zu einer Nukleinsäuresequenz innerhalb des selbst-stabilisierten Oligonukleotids umfaßt,
wobei entweder die Oligonukleotidsequenz der selbst-komplementären Region oder die komplementäre Nukleinsäuresequenz innerhalb des selbst-stabilisierten Oligonukleotids den größten Teil der am 3'-Ende gelegenen Nukleotide im selbst-stabilisierten Oligonukleotid umfaßt,
wobei die Oligonukleotidsequenz der selbst-komplementären Region und die komplementäre Nukleinsäuresequenz innerhalb des selbst-stabilisierten Oligonukleotids einen stabilen Doppelstrang unter physiologischen Bedingungen bilden,
mit der Maßgabe, daß kein Teil der Ziel-hybridisierenden Region zwischen der Oligonukleotidsequenz der selbst-komplementären Region und der komplementären Nukleinsäuresequenz innerhalb des selbst-stabilisierten Oligonukleotids ist,
mit der weiteren Maßgabe, daß die Ziel-hybridisierende Region nicht das gesamte Molekül umfaßt und
mit der weiteren Maßgabe, daß die Ziel-hybridisierende Region und die komplementäre Nukleinsäuresequenz der selbst-komplementären Region innerhalb des selbst-stabilisierten Oligonukleotids mindestens ein gemeinsames Nukleotid besitzen.

2. Selbst-stabilisiertes Oligonukleotid nach Anspruch 1, wobei die Ziel-hybridisierende Region vier oder mehr aufeinanderfolgende Desoxyribonukleotid-phosphodiester-, -phosphorothioat- oder -phosphorodithioat-Gruppen einschließt.

3. Selbst-stabilisiertes Oligonukleotid nach Anspruch 1 oder 2, wobei die selbst-komplementäre Region Nukleotide umfaßt ausgewählt aus der Gruppe bestehend aus Desoxyribonukleotid- oder Ribonukleotid-phosphodiester-, -phosphotriester-, -phosphorothioat-, -phosphorodithioat-, -phosphoroamidat-, -alkylphosphonat- und -alkylphosphonothioat-Gruppen.

4. Selbst-stabilisiertes Oligonukleotid nach einem der Ansprüche 1 bis 3, wobei das Virus ausgewählt ist aus der Gruppe bestehend aus HIV, Herpes simplex Virus, humanem Papillomavirus, Influenzavirus, Maul- und Klauenseuche-Virus, Gelbfieber-Virus, Varicella-Zoster-Virus und Cucumber-Mosaic-Virus (Gurken-Mosaik-Virus),
wobei der pathogene Organismus ausgewählt ist aus der Gruppe bestehend aus *Plasmodium falciparum, Trypanosoma brucei, Leishmania* und *Fasciola hepatica* und
wobei das zelluläre Gen ausgewählt ist aus einem Gen kodierend für ein Prion-Protein oder ein Amyloid-artiges Protein der Alzheimerschen Krankheit, aus Onkogenen oder aus Proto-Onkogenen.

5. Selbst-stabilisiertes Oligonukleotid nach einem der Ansprüche 1 bis 4, wobei die Ziel-hybridisierende Region die Oligonukleotidsequenz von etwa 8 bis etwa 50 Nukleotide umfaßt und die selbst-komplementäre Region die Oligonukleotidsequenz von 6, 8 oder etwa 10 Nukleotide umfaßt.

6. Selbst-stabilisiertes Oligonukleotid nach Anspruch 5, wobei die selbst-komplementäre Region die Oligonukleotidsequenz von etwa 10 Nukleotiden umfaßt.

7. In-vitro Verfahren zur Hemmung der Genexpression eines Virus, eines pathogenen Organismus oder eines zellulären Gens umfassend
Kontaktieren eines selbst-stabilisierten Oligonukleotids an Virus- oder Pathogen-infizierte Zellen oder an nicht-infizierte Zellen, wobei das selbst-stabilisierte Oligonukleotid eine Ziel-hybridisierende Region und eine selbst-komplementäre Region umfaßt,
wobei die Ziel-hybridisierende Region eine Oligonukleotidsequenz von mindestens 8 Nukleotiden komplementär zu einer Nukleinsäuresequenz eines Virus, eines pathogenen Organismus oder eines zellulären Gens umfaßt,
wobei die selbst-komplementäre Region eine Oligonukleotidsequenz von mindestens vier Nukleotiden komplementär zu einer Nukleinsäuresequenz innerhalb des selbst-stabilisierten Oligonukleotids umfaßt,
wobei entweder die Oligonukleotidsequenz der selbst-komplementären Region oder die komplementäre Nukleinsäuresequenz innerhalb des selbst-stabilisierten Oligonukleotids den größten Teil der am 3'-Ende gelegenen Nukleotide im selbst-stabilisierten Oligonukleotid umfaßt,
wobei die Oligonukleotidsequenz der selbst-komplementären Region und die komplementäre Nukleinsäuresequenz innerhalb des selbst-stabilisierten Oligonukleotids einen stabilen Doppelstrang unter physiologischen Bedingungen bilden,
mit der Maßgabe, daß kein Teil der Ziel-hybridisierenden Region zwischen der Oligonukleotidsequenz der selbst-komplementären Region und der komplementären Nukleinsäuresequenz innerhalb des selbst-stabilisierten Oligonukleotids ist,
mit der weiteren Maßgabe, daß die Ziel-hybridisierende Region nicht das gesamte Molekül umfaßt und
mit der weiteren Maßgabe, daß die Ziel-hybridisierende Region und die komplementäre Nukleinsäuresequenz der selbst-komplementären Region innerhalb des selbst-stabilisierten Oligonukleotids mindestens ein gemeinsames Nukleotid besitzen.

8. Verfahren nach Anspruch 7, wobei die selbst-komplementäre Region die Oligonukleotidsequenz von etwa 10 Nukleotiden komplementär zu einer Nukleinsäuresequenz innerhalb des selbst-stabilisierten Oligonukleotids umfaßt.

9. Verfahren nach Anspruch 7 oder 8, wobei die Ziel-hybridisierende Region vier oder mehr aufeinanderfolgende Desoxyribonukleotid-phosphodiester-, -phosphorothioat- oder -phosphorodithioat-Gruppen einschließt.

10. Verfahren nach einem der Ansprüche 7 bis 9, wobei das Virus ausgewählt ist aus der Gruppe bestehend aus HIV, Herpes simplex Virus, humanem Papillomavirus, Influenzavirus, Maul- und Klauenseuche-Virus, Gelbfieber-Virus, Varicella-Zoster-Virus und Cucumber-Mosaic-Virus (Gurken-Mosaik-Virus),
wobei der pathogene Organismus ausgewählt ist aus der Gruppe bestehend aus *Plasmodium falciparum, Trypanosoma brucei, Leishmania* und *Fasciola hepatica* und
wobei das zelluläre Gen ausgewählt ist aus einem Gen kodierend für ein Prion-Protein oder ein Amyloid-artiges Protein der Alzheimerschen Krankheit, aus Onkogenen oder aus Proto-Onkogenen.

11. Verwendung eines selbst-stabilisierten Oligonukleotids nach einem der Ansprüche 1 bis 6 zur Herstellung eines Arzneimittels zur Hemmung der Genexpression eines Virus, eines pathogenen Organismus oder eines zellulären Gens.

## Revendications

1. Oligonucléotide auto-stabilisé, comprenant une région cible d'hybridation et une région auto-complémentaire,
dans lequel la région cible d'hybridation comprend une séquence oligonucléotidique d'au moins 8 nucléotides qui est complémentaire d'une séquence d'acide nucléique provenant d'un virus, d'un organisme pathogène ou d'un gène cellulaire,
dans lequel la région auto-complémentaire comprend une séquence oligonucléotidique d'au moins 4 nucléotides qui est complémentaire d'une séquence d'acide nucléique contenue dans l'oligonucléotide auto-stabilisé,
dans lequel soit la séquence oligonucléotidique de la région auto-complémentaire, soit la séquence d'acide nucléique complémentaire contenue dans l'oligonucléotide auto-stabilisé, comprend la plus grande partie de la séquence nucléotidique en 3' dans l'oligonucléotide auto-stabilisé,
dans lequel la séquence oligonucléotidique de la région auto-complémentaire, et la séquence d'acide nucléique complémentaire contenue dans l'oligonucléotide auto-stabilisé, forment un duplex stable dans des conditions physiologiques,
à condition qu'aucune portion de la région cible d'hybridation ne soit située entre la séquence oligonucléotidique de la région auto-complémentaire et la séquence d'acide nucléique complémentaire contenue dans l'oligonucléotide auto-stabilisé,
à condition en outre que la région cible d'hybridation ne comprenne pas la molécule entière, et
à condition en outre que la région cible d'hybridation et que la séquence d'acide nucléique complémentaire de la région auto-complémentaire contenue dans l'oligonucléotide auto-stabilisé aient au moins un nucléotide en commun.

2. Oligonucléotide auto-stabilisé selon la revendication 1, dans lequel la région cible d'hybridation comporte quatre ou plus désoxyribonucléotides -phosphodiesters, -phosphorothioates, ou -phosphorodithioates, contigus.

3. Oligonucléotide auto-stabilisé selon la revendication 1 ou 2, dans lequel la région auto-complémentaire contient des nucléotides choisis dans le groupe constitué de désoxyribonucléotides ou ribonucléotides -phosphodiesters, -phosphotriesters, -phosphorothioates, -phosphorodithioates, -phosphoramidates, -alkylphosphonates, et -alkylphosphonothioates.

4. Oligonucléotide auto-stabilisé selon l'une quelconque des revendications 1 à 3,
dans lequel le virus est choisi dans le groupe constitué des : virus de l'immunodéficience humaine, virus de l'herpès simplex, virus du papillome humain, virus de la grippe, virus de la fièvre aphteuse, virus de la fièvre jaune, virus varicelle-zona, et virus de la mosaïque du concombre,
dans lequel l'organisme pathogène est choisi dans le groupe constitué de *Plasmodium falciparum, Trypanosoma brucei, Leishmania,* et *Fasciola hepatica,* et
dans lequel le gène cellulaire est choisi parmi un gène codant pour une protéine prion ou une protéine du type amyloïde de la maladie d'Alzheimer ou parmi les oncogènes ou les proto-oncogènes.

5. Oligonucléotide auto-stabilisé selon l'une quelconque des revendications 1 à 4, dans lequel la région cible d'hybridation comprend ladite séquence oligonucléotidique composée d'environ 8 à environ 50 nucléotides, et la région auto-complémentaire comprend ladite séquence oligonucléotidique composée de 6, 8 ou environ 10 nucléotides.

6. Oligonucléotide auto-stabilisé selon la revendication 5, dans lequel ladite région auto-complémentaire comprend ladite séquence oligonucléotidique composée d'environ 10 nucléotides.

7. Procédé in vitro pour inhiber l'expression génique d'un virus, d'un organisme pathogène, ou d'un gène cellulaire, consistant à fournir un oligonucléotide auto-stabilisé à des cellules infectées par un virus ou un pathogène, ou à des cellules non infectées, respectivement, l'oligonucléotide auto-stabilisé comprenant une région cible d'hybridation et une région auto-complémentaire,
dans lequel la région cible d'hybridation comprend une séquence oligonucléotidique d'au moins 8 nucléotides qui est complémentaire d'une séquence d'acide nucléique provenant d'un virus, d'un organisme pathogène ou d'un gène cellulaire,
dans lequel la région auto-complémentaire comprend une séquence oligonucléotidique d'au moins 4 nucléotides qui est complémentaire d'une séquence d'acide nucléique contenue dans l'oligonucléotide auto-stabilisé,
dans lequel soit la séquence oligonucléotidique de la région auto-complémentaire, soit la séquence d'acide nucléique complémentaire contenue dans l'oligonucléotide auto-stabilisé, comprend la plus grande partie de la séquence nucléotidique en 3' dans l'oligonucléotide auto-stabilisé,
dans lequel la séquence oligonucléotidique de la région auto-complémentaire, et la séquence d'acide nucléique complémentaire contenue dans l'oligonucléotide auto-stabilisé, forment un duplex stable dans des conditions physiologiques,
à condition qu'aucune portion de la région cible d'hybridation ne soit située entre la séquence oligonucléotidique de la région auto-complémentaire et la séquence d'acide nucléique complémentaire contenue dans l'oligonucléotide auto-stabilisé,
à condition en outre que la région cible d'hybridation ne comprenne pas la molécule entière, et
à condition en outre que la région cible d'hybridation et que la séquence d'acide nucléique complémentaire de la région auto-complémentaire contenue dans l'oligonucléotide auto-stabilisé aient au moins un nucléotide en commun.

8. Procédé selon la revendication 7, dans lequel la région auto-complémentaire comprend ladite séquence oligonucléotidique d'environ 10 nucléotides qui est complémentaire d'une séquence d'acide nucléique contenue dans l'oligonucléotide auto-stabilisé.

9. Procédé selon la revendication 7 ou 8, dans lequel la région cible d'hybridation comporte 4 ou plus désoxyribonucléotides -phosphodiesters, -phosphorothioates, ou -phosphorodithioates, contigus.

10. Procédé selon l'une quelconque des revendications 7 à 9,
dans lequel le virus est choisi dans le groupe constitué des virus de l'immunodéficience humaine, virus de l'herpès simplex, virus du papillome humain, virus de la grippe, virus de la fièvre aphteuse, virus de la fièvre jaune, virus varicelle-zona, et virus de la mosaïque du concombre,
dans lequel l'organisme pathogène est choisi dans le groupe constitué de *Plasmodium falciparum, Trypanosoma brucei, Leishmania,* et *Fasciola hepatica,* et
dans lequel le gène cellulaire est choisi parmi un gène codant pour une protéine prion ou une protéine du type amyloïde de la maladie d'Alzheimer ou parmi les oncogènes ou les proto-oncogènes.

11. Utilisation d'un oligonucléotide auto-stabilisé selon l'une quelconque des revendications 1 à 6 pour la préparation d'une composition pharmaceutique destinée à inhiber l'expression génique d'un virus, d'un organisme pathogène, ou d'un gène cellulaire.


### SEQUENCE LISTING

(1)GENERAL INFORMATION:
   (i)APPLICANT:
      (A) NAME: Hybridon
      (B) STREET: One Innovation Drive
      (C) CITY: Worchester
      (D) STATE: Massachusetts
      (E) COUNTRY: United States of America
      (F) ZIP: 01605
   (ii) TITLE OF INVENTION: Self-Stabilized Oligonucleotides As Novel Therapeutic Agents
   (iii) NUMBER OF SEQUENCES: 13
   (iv) COMPUTER READABLE FORM:
      (A)MEDIUM TYPE: Diskette, 3.5 in., DS, 1.44 MB
      (B)COMPUTER: IBM PC compatible
      (C) OPERATING SYSTEM: PC-DOS/MS-DOS
      (D)SOFTWARE: PatentIn Release #1.0, Version #1.25 (amendments performed in WordPerfect 5.1)
   (v)CURRENT APPLICATION DATA:
      (A)APPLICATION NUMBER: PCT/US93/06326
(2)INFORMATION FOR SEQ ID NO:1:
   (i) SEQUENCE CHARACTERISTICS:
      (A)LENGTH: 25 base pairs
      (B)TYPE: nucleic acid
      (C) STRANDEDNESS: single
      (D) TOPOLOGY: linear
   (ii)MOLECULE TYPE: DNA
   (iii)HYPOTHETICAL: NO
   (iv)ANTI-SENSE: YES
   (xi)SEQUENCE DESCRIPTION: SEQ ID NO: 1:
   CTCTCGCACC CATCTCTCTC CTTCT 25
(2)INFORMATION FOR SEQ ID NO:2:
   (i) SEQUENCE CHARACTERISTICS:
      (A)LENGTH: 35 base pairs
      (B)TYPE: nucleic acid
      (C) STRANDEDNESS: single
      (D) TOPOLOGY: linear
      (ii)MOLECULE TYPE: DNA
      (iii)HYPOTHETICAL: NO
      (iv)ANTI-SENSE: YES
      (xi)SEQUENCE DESCRIPTION: SEQ ID NO:2:
   CTCTCGCACC CATCTCTCTC CTTCTGGAGA GAGAT 35
(2)INFORMATION FOR SEQ ID NO:3:
   (i) SEQUENCE CHARACTERISTICS:
      (A)LENGTH: 36 base pairs
      (B)TYPE: nucleic acid
      (C)STRANDEDNESS: single
      (D) TOPOLOGY: linear
   (ii)MOLECULE TYPE: DNA
   (iii)HYPOTHETICAL: NO
   (iv)ANTI-SENSE: YES
   (xi)SEQUENCE DESCRIPTION: SEQ ID NO:3:
   CTCTCGCACC CATCTCTCTC CTTCTGGGTG CGAGAG 36
(2)INFORMATION FOR SEQ ID NO:4:
   (i) SEQUENCE CHARACTERISTICS:
      (A)LENGTH: 33 base pairs
      (B)TYPE: nucleic acid
      (C)STRANDEDNESS: single
      (D)TOPOLOGY: linear
   (ii)MOLECULE TYPE: DNA
   (iii)HYPOTHETICAL: NO
   (iv)ANTI-SENSE: YES
   (xi)SEQUENCE DESCRIPTION: SEQ ID NO:4:
   CTCTCGCACC CATCTCTCTC CTTCTGGAGA GAG 33
(2)INFORMATION FOR SEQ ID NO:5:
   (i) SEQUENCE CHARACTERISTICS:
      (A)LENGTH: 31 base pairs
      (B)TYPE: nucleic acid
      (C) STRANDEDNESS: single
      (D) TOPOLOGY: linear
   (ii)MOLECULE TYPE: DNA
   (iii)HYPOTHETICAL: NO
   (iv)ANTI-SENSE: YES
   (xi)SEQUENCE DESCRIPTION: SEQ ID NO:5:
   CTCTCGCACC CATCTCTCTC CTTCTGGAGA G 31
(2)INFORMATION FOR SEQ ID NO:6:
   (i) SEQUENCE CHARACTERISTICS:
      (A)LENGTH: 29 base pairs
      (B)TYPE: nucleic acid
      (C) STRANDEDNESS: single
      (D)TOPOLOGY: linear
   (ii) MOLECULE TYPE: DNA
   (iii)HYPOTHETICAL: NO
   (iv)ANTI-SENSE: YES
   (xi)SEQUENCE DESCRIPTION: SEQ ID NO:6:
   CTCTCGCACC CATCTCTCTC CTTCTGGAG 29
(2)INFORMATION FOR SEQ ID NO:7:
   (i) SEQUENCE CHARACTERISTICS:
      (A)LENGTH: 36 base pairs
      (B) TYPE: nucleic acid
      (C) STRANDEDNESS: single
      (D) TOPOLOGY: linear
   (ii)MOLECULE TYPE: DNA
   (iii)HYPOTHETICAL: NO
   (iv)ANTI-SENSE: YES
   (xi)SEQUENCE DESCRIPTION: SEQ ID NO:7:
   CTCTCGCACC CATCTCTCTC CTTCTTTTTT TTTTTT 36
(2)INFORMATION FOR SEQ ID NO:8:
   (i) SEQUENCE CHARACTERISTICS:
      (A)LENGTH: 35 base pairs
      (B)TYPE: nucleic acid
      (C)STRANDEDNESS: single
      (D) TOPOLOGY: linear
   (ii)MOLECULE TYPE: DNA
   (iii)HYPOTHETICAL: NO
   (iv)ANTI-SENSE: YES
   (xi)SEQUENCE DESCRIPTION: SEQ ID NO:8:
   CTCTCGCACC CATCTCTCTC CTTCTAGCCT CCGCT 35
(2)INFORMATION FOR SEQ ID NO:9:
   (i) SEQUENCE CHARACTERISTICS:
      (A)LENGTH: 38 basc pairs
      (B)TYPE: nucleic acid
      (C) STRANDEDNESS: single
      (D) TOPOLOGY: linear
   (ii)MOLECULE TYPE: RNA (genomic)
   (iii)HYPOTHETICAL: NO
   (iv)ANTI-SENSE: NO
   (xi)SEQUENCE DESCRIPTION: SEQ ID NO:9:
   AGGCTAGAAG GAGAGAGATG GGTGCGAGAG CGTCAGTA 38
(2)INFORMATION FOR SEQ ID NO:10:
   (i) SEQUENCE CHARACTERISTICS:
      (A)LENGTH: 30 base pairs
      (B) TYPE: nucleic acid
      (C) STRANDEDNESS: single
      (D) TOPOLOGY: linear
   (ii)MOLECULE TYPE: DNA
   (iii)HYPOTHETICAL: NO
   (iv)ANTI-SENSE: YES
   (xi)SEQUENCE DESCRIPTION: SEQ ID NO:10:
   CTCTCGCACC CATCTCTCTC CTTCTGGAGA 30
(2)INFORMATION FOR SEQ ID NO:11:
   (i) SEQUENCE CHARACTERISTICS:
      (A)LENGTH: 27 base pairs
      (B)TYPE: nucleic acid
      (C)STRANDEDNESS: single
      (D) TOPOLOGY: linear
   (ii) MOLECULE TYPE: DNA
   (iii)HYPOTHETICAL: NO
   (iv)ANTI-SENSE: YES
   (xi)SEQUENCE DESCRIPTION: SEQ ID NO:11:
   CTCTCGCACC CATCTCTCTC CTTCTGG 27
(2)INFORMATION FOR SEQ ID NO:12:
   (i)SEQUENCE CHARACTERISTICS:
      (A)LENGTH: 39 base pairs
      (B)TYPE: nucleic acid
      (C)STRANDEDNESS: single
      (D) TOPOLOGY: linear
   (ii)MOLECULE TYPE: RNA (genomic)
   (iii)HYPOTHETICAL: NO
   (iv)ANTI-SENSE: NO
   (xi)SEQUENCE DESCRIPTION: SEQ ID NO:12:
   AGAAGGAGAG AGAUGGGUGC GAGAGCGUCA GUAUUAAGC 39
(2)INFORMATION FOR SEQ ID NO:13:
   (i) SEQUENCE CHARACTERISTICS:
      (A)LENGTH: 53 base pairs
      (B)TYPE: nucleic acid
      (C)STRANDEDNESS: single
      (D)TOPOLOGY: linear
   (ii)MOLECULE TYPE: RNA
   (iii)HYPOTHETICAL: NO
   (iv)ANTI-SENSE: YES
   (xi)SEQUENCE DESCRIPTION: SEQ ID NO:13:
   GCUUAAUACU CUGAUGAGUC CGUGAGGACG AAACGCUCUC GCACUUGUGC GAG 53